# EUROPEAN PATENT APPLICATION

(11) **EP 4 353 740 A1**
(43) Date of publication of application: **17.04.2024**
(21) Application number: 22201482.1
(22) Date of filing: 13.10.2022
(51) Int. Cl.: C07K 14/37, C12N 15/82

(54) **CONSTRUCT FOR INDUCING SILENCING IN PLANTS AND METHOD FOR PROTECTING THEM AGAINST ANTHRACNOSE DISEASE**

(30) Priority: 12.10.2022 PT 2022118252
(71) Applicant: Universidade de Évora, 7000-803 Évora (PT)
(72) Inventor: DE QUEIROZ MATERATSKI, PATRICK JOSÉ, ÉVORA (PT); REIS VARANDA, CARLA MARISA, ÉVORA (PT); MURTEIRA RICO DA COSTA CAMPOS, MARIA DOROTEIA, ÉVORA (PT); CARDOSO PATANITA, MARIANA, BEJA (PT); BARRETO ALBUQUERQUE, ANDRÉ FILIPE, ÉVORA (PT); AMARO RIBEIRO, JOANA, REGUENGOS DE MONSARAZ (PT); FERNANDES FÉLIX, MARIA DO ROSÁRIO, ÉVORA (PT)
(74) Representative: Pereira da Cruz, Joao

(57) **Abstract**

The present invention discloses an isolated sequence having at least 95% homology with SEQ ID No 1, preferably at least 98% homology, even more preferably having at least 99% homology, the isolated sequence comprising at least one point mutation selected from: adenine to thymine at nucleotide 3200, cytosine to guanine at nucleotide 3361, guanine to cytosine at nucleotide 3365 and/or guanine to thymine at nucleotide 3368. The invention also relates to a process for silencing a target gene in a plant comprising the steps of i) obtaining a construct according to the previous claim; ii) introducing the construct inside the plant, preferably by using viral suspensions; inoculating into plants, preferably by spraying plants, even more preferably by spraying plants with a compressor set comprising an airbrush; iii) activating the plant's specific defence mechanism, consequently silencing the target gene. The present invention also discloses a composition for plant disease management and/or plant immunization.

## Description

### Field of the Invention

The present invention is enclosed in the area of Genetic Engineering and Biotechnology.

This invention relies on the triggering of Colletotrichum spp. silencing mechanism through 'Virus induced gene silencing' (VIGS). VIGS allows specific silencing of foreign genes that can be inserted in an optimized virus vector and then inoculated in plants. When a dsRNA sequence of a gene essential for the fungus survival is introduced into a plant through a virus vector, and the fungus "feeds" on that plant, it ingests the plant cytoplasm and acquires the dsRNA; RNAi will be triggered, homologous RNAs will be targeted, the expression of that fungal gene will be prevented and fungus will be destroyed, providing plants protection against the anthracnose disease.

### Background of the Invention

*Colletotrichum spp.* is the causal agent of anthracnose in olive trees, a very common and severe disease in Portugal. This disease has also a large impact in other Mediterranean countries such as Spain, Italy, and Serbia and Montenegro, and central and western Mediterranean countries, Australia and South Africa, as well as in other crops. Although the disease is distributed along all Mediterranean Basin it is in Portugal that it has a great expression, mainly due to the Atlantic influence (higher moisture during autumn) (Talhinhas, et al., 2005, 2009; Materatski, et al., 2018, 2019). The disease in Portugal, causes up to 100% losses, particularly higher in the widely cultivated varieties 'Galega vulgar', 'Cobrançosa', 'Maçanilha de Tavira' and lower in 'Azeiteira', 'Blanqueta', 'Negrinha de Freixo' and 'Picual' (Materatski, et al., 2018).

Symptoms typically occur on mature fruits (during autumn), as dark necrosis with abundant orange conidial masses, leading to premature fruit drop or mummification, damaged fruits and poor oil quality (high acidity) (Talhinhas, et al., 2009).

Under favorable conditions, symptoms can also occur on branches and leaves, leading to necrosis, severe defoliation and death of branches (Talhinhas, et al., 2006). As mentioned, the Portuguese olive cultivar, 'Galega vulgar', is very susceptible to anthracnose but the disease can also affect severely less susceptible varieties if environmental conditions are favorable.

Losses were reported from cultivars such as Arbequina and Picual, previously regarded as moderately resistant, and widely cultivated through-out Iberian Peninsula (Talhinhas, et al., 2005). This disease severely attacks other agriculturally important hosts including many dicotyledonous plants such as strawberry, apple, citrus, and stone fruits, because of this, it is considered a worldwide problem (Talhinhas, et al., 2006, 2009; Materatski, et al., 2018, 2019).

Because the issue of biosafety of transgenic plants is a growing concern, the present invention is particularly relevant as RNAi is used as a plant protection technique against *Colletotrichum spp.* without the need of producing transgenic plants. The introduction of dsRNA in plants is performed using a transient system involving a transformed plant virus as vector for silencing.

The high number of varieties of olive and the efficiency of the causal agents of diseases such as *Colletotrichum spp.* makes the goal of the present invention of great importance.

Until this date, the best control strategy for this fungal disease is often a complex process, it relies on synthetic fungicides that are unspecific, inefficient, expensive and harmful to the environment, leaving eradication or prevention through sanitary measures as important control strategies.

The present solution intended to innovatively overcome such issues and provide a unique solution to silence the disease, avoiding the existence of an infection of the plant.

### General Description

It is therefore an object of the present invention to provide an effective product and method to prevent and treat plants from Anthracnose disease.

The present invention discloses a vector, which is plant virus-based, modified to carry and express a partial sequence of the β-tubulin 2 gene of the fungi *Colletotrichum spp.* in olive plants.

When *Colletotrichum spp.,* during its life cycle and infection, ingests the plant cytoplasm it acquires the dsRNA of the β-tubulin gene, RNAi will be triggered inside the fungus, homologous RNAs will be targeted, the expression of that fungal gene will be prevented and fungus will be destroyed, providing plants protection against the anthracnose disease. The vector will be capable of replicating in several plants and highly efficient in silencing, acting as a VIGS vector.

In an embodiment, the construct carries a partial β-tubulin 2 gene sequence in antisense orientation.

In an embodiment, the construct is introduced inside olive plants without causing them any viral symptoms. This will activate silencing, after its ingestion by the fungus, as fungus will recognize the sequences and activate its specific defence mechanism, causing its self-destruction.

In an embodiment, the present disclosure is related to an isolated nucleic acid sequence having at least 95% homology with SEQ ID No 1, preferably at least 98% homology, even more preferably having at least 99% homology, the isolated sequence comprising at least one point mutation selected from: adenine to thymine at nucleotide 3200, cytosine to guanine at nucleotide 3361, guanine to cytosine at nucleotide 3365 and/or guanine to thymine at nucleotide 3368.

In a further embodiment, an isolated sequence is disclosed, wherein the sequence is a partial sequence of Olive mild mosaic virus (OMMV).

In a further embodiment, a vector comprising the isolated sequence of the present invention is disclosed.

In a further embodiment, a construct comprising the vector of the present invention and a partial isolated sequence of β-tubulin 2 gene of a *Colletotrichum* species, preferably *Colletotrichum acutatum,* in antisense orientation, is disclosed .

In a further embodiment, a construct comprising the vector of the present invention and an isolated sequence having at least 95% homology with SEQ ID No 2, preferably at least 98% homology, even more preferably having at least 99% homology with SEQ ID No 2, is diclosed.

In a further embodiment, a construct comprising an isolated sequence having at least 95% homology with SEQ ID No 3, preferably at least 98% homology, even more preferably having at least 99% homology with SEQ ID No 3, is disclosed.

In a further embodiment, it is disclosed a process for silencing a target gene in a plant comprising the steps of:
i) obtaining a vector or a construct according to any of the present invention;
ii) introducing said vector or construct inside the plant, preferably by using viral suspension;
iii) inoculating the vector or construct into the plant or fruit, preferably by spraying, even more preferably by spraying with a compressor comprising an airbrush;
consequently activating the plant's specific defence mechanism, therefore silencing the target gene.

In a further embodiment, a process according to the present invention is disclosed, wherein the plant is an olive tree, particularly *Olea europaea,* a mature fruit, branches or leaves of a plant.

In a particular embodiment, a process for producing and maintaining the vector of the present invention in *Nicotiana benthamiana* is disclosed.

In a further embodiment, a plant comprising a sequence with at least 95% homology, preferably at least 98% homology, even more preferably at least 99% homology with SEQ ID No.1, SEQ ID No 2, SEQ ID No 3 or a combination thereof, wherein the plant is not obtained exclusively by crossing and selection, is disclosed.

The present invention also discloses a plant according to the previous embodiment, wherein the plant is an olive tree, particularly *Olea europaea.*

In a further embodiment, the present disclosure encompasses a composition, particularly a phytopharmaceutical composition, for plant treatment comprising the sequence, vector or construct of any of the embodiments of the present invention.

In a further embodiment, the present disclosure encompasses the composition of the previous embodiment for Plant disease management and/or Plant immunization.

### Detailed Description of the Invention

The more general and advantageous configurations of the present invention are described above. Such configurations are detailed below in accordance with other advantageous and/or preferred embodiments of implementation of the present invention.

### Example 1

The virus vector is based on Olive mild mosaic virus (OMMV), a virus originally isolated from olive (Cardoso et al., 2005). An infectious transcript of OMMV cDNA clone obtained from OMMV type strain (Accession number HQ651834.1) was modified to be non-fungal transmitted, through a single mutation from adenine to thymine at nucleotide (nt) 3200 resulting in a non-synonymous change from asparagine to tyrosine at position 189 of the coat protein (CP) (Varanda et al., 2011). This virus mutant was further modified to cause no disease symptoms in plants, through 3-point mutations: cytosine to guanine at nt 3361 (proline to alanine at position 242 of CP aa sequence); guanine to cytosine at nt 3365 (alanine to proline at position 244 of CP aa sequence) and guanine to thymine at nt 3368 (alanine to serine at position 245 of CP aa sequence). This new virus mutant was named OMMV_noSnoT (SEQ ID No 1).

The 3' sequence of the β-tubulin 2 gene of *Colletotrichum acutatum* (SEQ ID No 2, Accession number AJ748605.1, nucleotide 1 to 550) 550 nt in length, was amplified through Polymerase chain reaction using primers Beta1_5' (5' GGTAACCAGATTGGTGCTGC 3') and Beta550_3' (5' GCAGTCGCAGCCCTCAGCCT 3') and the KOD Hot Start DNA Polymerase that inhibits the 3' - 5' exonuclease activity, producing blunt-ended DNA products.

This sequence (SEQ ID No 2) was then ligated with T4 DNA ligase into OMMV_noSnoT (SEQ ID No 1), previously digested with the restriction enzyme HPal, that cuts in the position nt3454, 9 nt downstream of the CP and leaves blunt ends. Sequencing was performed to select constructs carrying β-tubulin 2 gene sequence in antisense orientation.

The vector presented, consisting of OMMV_noSnoT carrying a partial sequence of β-tubulin 2 gene, is here termed GaFree vector (SEQ ID No. 3).

GaFree (SEQ ID No 3) vector is stable, it replicates and accumulates in olive plants, causing no disease symptoms and is able to produce dsRNA sequence of a β-tubulin 2 gene essential for the *Colletotrichum spp.* survival conferring protection of olive plants against the anthracnose disease.

In an embodiment, the virus-based vector described in the previous section, for conferring protection of plants against *Colletotrichum spp.* in olive plants is produced and maintained in *Nicotiana benthamiana* plants.

in an embodiment, macerated leaves are subjected to viral purification as described in Varanda et al., 2011, the disclosure being herein incorporated by reference.

In an embodiment, viral suspensions are used to spray inoculate plants, after loading the mixture into an airbrush attached to a compressor set which is used to spray plant leaves.

In an embodiment, mutations performed to create OMMV_noSnoT were introduced using the QuikChange Site-Directed Mutagenesis kit Stratagene, in accordance to the instruction manual. Primers were designed according to the primer design guidelines and were as follows: Mut_OMMV_Fwd: 5' GCTGGGGACGGCGGAGCTGTGCCTTCCACAGCTGTGGGC 3' and Mut_OMMV_Rev: 5' GCCCACAGCTGTGGAAGGCACAGCTCCGCCGTCCCCAGC; mutations are underlined. Mutation strand synthesis reaction was, for a final volume of 50 µL, as follows: 5 µL of 10x reaction buffer, 50 ng of plasmid DNA (OMMV in puC18), 125 ng of Mut_OMMV_Fwd and 125 ng of Mut_OMMV_Rev, 1 µL of dNTP mix and 2,5 U of PfuTurbo DNA polymerase. Cycling parameters consisted of: 1 cycle at 95 ºC for 30 seconds; 18 cycles of 95 °C for 45 seconds, 55 ºC for 1 minute and 68 °C for 6 minutes. Reaction was placed on ice for 2 minutes to cool the reaction. Digestion of amplification products was performed through Dpn I digestion to digest the parental dsDNA. 1 µL Dpn I restriction enzyme was added to the reaction and incubated at 37 °C for 1 hour. Transformation was performed using XL1-Blue Supercompetent cells following the manufacturer's instructions.

The amplification reaction for the 3' sequence of the β-tubulin 2 partial gene of *Colletotrichum acutatum* consisted of 50 µL of: 1X buffer for KOD Start DNA Polymerase, 1,5 mM of MgSO4, 0,2 mM of each dNTP, 0,3 µM of primer Beta1_5', 0,3 µL of primer Beta550_3', 1U KOD Hot Start DNA Polymerase and 10 ng of plasmid DNA of Colletotrichum acutatum. Cycling conditions consisted of one cycle at 95 °C for 2 min; 40 cycles of 95 °C for 40 seconds, 58 °C for 55 seconds and 72 °C for 2 minutes, and then 72 °C for 10 minutes for extension.

In an embodiment, constructs were expressed through inoculation, followed by inoculation with *Colletotrichum acutatum* isolate after 24h to 72h, preferably 48h. Inoculated leaves were harvested 3 - 8 days, preferably 5 days after inoculation for RNA extraction. Constructs limited *Colletotrichum acutatum* isolate genomic RNA accumulation and propagation compared to no construct-inoculated plants.

As will be clear to one skilled in the art, the present invention should not be limited to the embodiments described herein, and a number of changes are possible which remain within the scope of the present invention.

Of course, the preferred embodiments shown above are combinable, in the different possible forms, being herein avoided the repetition all such combinations.

### REFERENCES

Cardoso JMS, Félix MR, Clara MIE, Oliveira S (2005) The complete genome sequence of a new necrovirus isolated from Olea europaea L. Archives of Virology 150:815-823
Varanda CMR, Félix MR, Soares CM, Oliveira S, Clara MIE (2011a) Specific amino acids of Olive mild mosaic virus coat protein are involved on transmission by Olpidium brassicae. Journal of General Virology 92:2209-2213.
Materatski, P; Varanda, C; Carvalho, T; Dias, AB; Campos, MD; Rei, F; Félix, MR (2018) Diversity of Colletotrichum Species Associated with Olive Anthracnose and New Perspectives on Controlling the Disease in Portugal. Agronomy. 8, 301; doi:10.3390/agronomy8120301
Materatski, P.; Varanda, C.; Carvalho, T.; Dias, A.B.; Campos, M.D.; Gomes, L.; Nobre, T.; Rei, F.; Félix, M.d.R. Effect of Long-Term Fungicide Applications on Virulence and Diversity of Colletotrichum spp. Associated to Olive Anthracnose. Plants 2019, 8, 311. https://doi.org/10.3390/plants8090311
Talhinhas, P., Sreenivasaprasad, S., Neves-Martins, J., & Oliveira, H. (2005). Molecular and phenotypic analyses reveal the association of diverse Colletotrichum acutatum groups and a low level of C. gloeosporioides with olive anthracnose. Applied and Environmental Microbiology, 71, 2987-2998
Talhinhas, P., Leitão, J. & Neves-Martins, J. Collection of Lupinus angustifolius L. Germplasm and Characterisation of Morphological and Molecular Diversity. Genet Resour Crop Evol 53, 563-578 (2006). https://doi.org/10.1007/s10722-004-2684-0
Talhinhas P., Neves-Martins J., OliveiraH. and Sreenivasaprasad S. (2009). The distinctive population structure of Colletotrichum species associated with olive anthracnose in the Algarve region of Portugal reflects a host-pathogen diversity hot spot. FEMS Microbiol Lett, 296, 31-38.

## Claims

1. Isolated nucleic acid sequence having at least 95% homology with SEQ ID No 1, preferably at least 98% homology with SEQ ID No 1, even more preferably having at least 99% homology with SEQ ID No 1, the isolated sequence comprising at least one point mutation selected from: adenine to thymine at nucleotide 3200, cytosine to guanine at nucleotide 3361, guanine to cytosine at nucleotide 3365 and/or guanine to thymine at nucleotide 3368.

2. Isolated sequence according to the previous claim wherein the sequence is a partial sequence of Olive mild mosaic virus (OMMV).

3. Vector comprising the isolated sequence of any of the previous claims.

4. Construct comprising the sequence or vector according to any of the previous claims and a partial isolated sequence of β-tubulin 2 gene of a *Colletotrichum* species, preferably *Colletotrichum acutatum,* in antisense orientation.

5. Construct comprising the sequence or vector according to claims 1-3 and an isolated sequence having at least 95% homology with SEQ ID No 2, preferably at least 98% homology with SEQ ID No 2, even more preferably having at least 99% homology with SEQ ID No 2.

6. Construct according to claim 4 comprising an isolated sequence having at least 95% homology with SEQ ID No 3, preferably at least 98% homology with SEQ ID No 3, even more preferably having at least 99% homology with SEQ ID No 3.

7. Process for silencing a target gene in a plant comprising the steps of:
i) obtaining a sequence, vector or a construct according to any of the previous claims;
ii) introducing said sequence, vector or construct inside the plant, preferably by using viral suspension;
iii) inoculating the sequence, vector or construct into the plant, preferably by spraying, even more preferably by spraying with a compressor comprising an airbrush;
consequently activating the plant's specific defence mechanism, therefore silencing the target gene.

8. Process according to the previous claim wherein the plant is an olive tree, particularly *Olea europaea,* a mature fruit, branches or leaves of a plant.

9. Process for producing and maintaining the vector of claims 1-3 in *Nicotiana benthamiana.*

10. Plant comprising a sequence with at least 95% homology, preferably at least 98% homology, even more preferably at least 99% homology with SEQ ID No.1, SEQ ID No 2, SEQ ID No 3 or a combination thereof, wherein the plant is not obtained exclusively by crossing and selection.

11. Plant according to the previous claim, wherein the plant is an olive tree, particularly *Olea europaea.*

12. Composition, particularly a phytopharmaceutical composition, for plant treatment comprising the sequence, vector or construct of claims 1-6.

13. Composition of the previous claim for Plant disease management and/or Plant immunization.
